# EUROPEAN PATENT APPLICATION

(11) **EP 4 066 824 A1**
(43) Date of publication of application: **05.10.2022**
(21) Application number: 21166461.0
(22) Date of filing: 31.03.2021
(51) Int. Cl.: A61K 31/085, A61K 31/138, A61K 31/352, A61K 31/353, A61K 31/4535, A61K 31/55, A61K 31/565, A61K 31/566, A61K 31/592, A61K 31/593, A61K 31/7048, A61K 39/215, A61K 45/06, A61P 31/16

(54) **COMBINATION THERAPY FOR COVID-19 VACCINATION**

(71) Applicant: Dompe' Farmaceutici S.P.A., 20122 Milan (IT)
(72) Inventor: ALLEGRETTI, Marcello, 67100 L'Auila (IT); BECCARI, Andrea Rosario, 80131 Napoli (IT); CESTA, Maria Candida, 67100 L'Aquila (IT); MAURI, Elisabetta Maria Ester, 20122 Milano (IT); TALARICO, Carmine, 80131 Napoli (IT)
(74) Representative: Mauri, Elisabetta Maria Ester

(57) **Abstract**

A combination of an estrogen receptor agonist and/or a vitamin D receptor agonist and a COVID-19 vaccine.

## Description

### FIELD OF THE INVENTION

The present invention relates to the combination of an estrogen receptor agonist and/or vitamin D receptor agonist with COVID-19 vaccination.

### STATE OF THE ART

Coronaviruses (Covs) are a large family of single-stranded, enveloped RNA viruses that belong to the Coronaviridae family. The limited number of coronaviruses known to be able to infect humans were considered in the past as relatively harmless respiratory human pathogens, causing mild infections. However, two coronavirus subtypes have emerged, Severe Acute Respiratory Syndrome Coronavirus (SARS-CoV) and the Middle East Respiratory Syndrome Coronavirus (MERS-CoV), that cause severe and sometimes fatal respiratory tract infections in humans (Pereira, H et al., 1989 Coronaviridae. Andrewes 'Viruses of Vertebrates, 5th ed.pp. 42-57, Holmes, K.V. et al., Virology 1996, 1: 1075-1093). In December 2019, atypical pneumonia cases occurred in China and the cause was later identified as being a novel coronavirus. The World Health Organization (WHO) named the virus as SARS-CoV-2 and the related disease as COVID-19.

The virus spread rapidly worldwide, and on 11 March 2020 the WHO declared SARS-CoV-2 infection as a pandemic. Most people infected with COVID-19 experience mild to moderate respiratory illness (fever, fatigue, dry cough and dyspnea) and recover without requiring special treatments. Older people, and those with underlying medical problems like cardiovascular disease, diabetes, chronic respiratory disease, and cancer are more likely to develop serious illness. Furthermore, analysis of epidemiological and clinical characteristics and outcomes of patients infected by SARS-CoV-2 have shown that 15% of people with symptomatic COVID-19 have significant disease including severe pneumonia, and 5% experience critical disease with life-threatening complications. Critical disease includes acute respiratory distress syndrome (ARDS), sepsis, septic shock, cardiac disease, thromboembolic events, such as pulmonary embolism and multi-organ failure.

There is also growing evidence that in those who develop critical COVID-19 disease, long-term consequences such as rare neurological and psychiatric complications are reasonably expected.These may include stroke, delirium, anxiety, depression, damage or inflammation of the brain and sleep disturbances.

Due to the dramatic rise in cases and deaths worldwide and the social and economic consequences thereof, efforts have focused to the development of a safe and effective vaccines to help control and bring to an end the COVID-19 pandemic.

Different vaccines are under development and some have already been approved for prevention of COVID-19. All COVID-19 vaccines and vaccine candidates act by inducing an immune response against virus protein antigens.

SARS-COV-2 is characterised by four structural proteins, namely spike (S) protein, envelope (E) protein, membrane (M) protein and nucleocapsid (N) protein (Chen Y et al, J Med Virol 2020, 92: 418-423).

Currently, the spike protein is the main protein used as a target antigen in COVID-19 vaccines. This protein is considered an important antigenic determinant capable of inducing a protective immune response (Ou et al, Nat Commun 2020; 11: 1620). Furthermore, it is an essential molecule for entry of the virus into cells through uses the cell entry receptor Angiotensin-converting enzyme II (ACE2). In particular, the S protein contains two subunits, S1, which mediate receptor binding to ACE2 through the receptor binding domain (RBD) and S2, which is responsible for membrane fusion (Letko et al, Nat Microbiol 2020, 5: 562-569). Therefore, antibodies targeting the S protein prevent the virus from entering and replicating inside the host cells.

The vaccines and vaccine candidates developed or under development for COVID-19 are mainly classified into two broad categories: traditional inactivated or live-attenuated virus vaccines, that introduce viral protein antigens into the body of the host and newer gene-based vaccines, such as DNA vaccines and mRNA vaccines.

DNA and mRNA vaccines deliver genes encoding the viral protein antigens for in vivo production in the cells of the host and are considered the best approach for COVID-19 vaccination compared to the traditional vaccine approaches, in view of their improved safety and suitability for mass manufacturing.

The three vaccines approved so far by EMA are all based on this technological approach: the mRNA based Comirnaty developed by Pfizer and BioNTech, the mRNA based COVID-19 Vaccine developed by Moderna and the DNA, viral vector based COVID-19 *ChAdOx1-S* vaccine developed by Oxford University and AstraZeneca.

The administration of these vaccines to a subject induces the production of spike protein that is released in the blood flow, causing the immune system to initiate an immune response.

Recent evidence has suggested a potential activity of the SARS-CoV-2 spike protein on cell signalling independent from facilitation of viral infection.

In particular, it has been shown that the full length S1 subunit of SARS-CoV-2 spike protein can induce pro-inflammatory responses *in vitro* in murine and human macrophages (Shirato et al, Heyon 2021, 7(2) e06187) and can activate cell signaling events in cultured human vascular cells (Suzuki et al, Vascular Pharmacology 137 (2021) 106823).

In view of the above, it therefore needs to be investigated whether the presence in the body of the spike protein produced by vaccination may interfere with biological processes, thereby hindering the immune response and/or causing side effects in the short and long term.

Steroid hormones have been shown to regulate a wide range of physiological functions such as development and maintenance of secondary sexual characteristics, metabolism, blood salt balance, immune and inflammatory responses, response to stress and/or neuronal function.

Many studies have demonstrated that both vitamin D and estrogen, in addition to their primary functions in the regulation of bone homeostasis and secondary sexual characteristics, respectively, play a key role in the regulation of inflammation and immune response to infection, and that this action may be particularly relevant in the reaction to COVID-19 infection.

In particular, many studies have shown that both these steroids are actively involved in the regulation of innate and adaptive immune responses and in the control of the cytokine storm induced in inflammatory conditions (Schwalfenberg et al, Mol Nutr Food Res 2011, 55(1): 96-108, Bilezikian et al, European Journal of Endocrinology 2020, 183: 133-147, Khanet al, Front Immunol 2015, 6: 635).

Evidence suggests that vitamin D and estrogen are key players for an effective immune response against COVID-19 and for controlling the associated damaging inflammatory response (Ohaegbulam et al, Am J Ther 2020, 27:e485-e490, Entrenas Castillo et al, J. Steroid Biochem Mol Biol 2020, 203:105751).

In particular, some retrospective studies have demonstrated that hypovitaminosis D may represent a risk factor for the ongoing epidemic of COVID-19 (Bilezikian et al, European Journal of Endocrinology 2020, 183: 133-147; D'Avolio et al, Nutrients 2020, 12: E1359). Furthermore, epidemiological data indicate that COVID-19 has a significantly higher lethality in men than in women (ratios up to 3:1), suggesting the presence of sex-dependent biological factors underlying these differences in disease outcome (Pagano et al, Trends in Endocrinology & Metabolism 2021, 32: 1).

The activity of these molecules is mediated by nuclear receptors, the estrogen and the vitamin D receptor, that function as hormone-activated transcription factors. Upon ligand activation, these receptors bind to the promoters of target genes and form a transcriptional complex with a number of interacting proteins, collectively known as coregulators, that can either activate (coactivators (CoA)) or inactivate (corepressors (CoR)) transcriptional activity, thereby triggering the activation or repression of target gene expression (Patel et al, Pharmacology & Therapeutics 2018, 186: 1-24; Zella et al, Arch Biochem Biophys 2007, 460(2): 206-212). The interaction between nuclear receptors and coactivators is mediated by an LxxLL motif (where L is leucine and x is any amino acid) contained and shared within the coactivator proteins, that is necessary and sufficient for the binding of these proteins to the receptor and for enhancing its transcriptional activity (Patel et al, Pharmacology & Therapeutics 2018, 186: 1-24; Zella et al, Arch Biochem Biophys 2007, 460(2): 206-212).

Although the activity of estrogen and Vitamin D receptor ligands on the respective receptors is expected, in terms of support to the human body for the development of the immune response, what is observed in COVID-19 patients is higher than expected.

### SUMMARY OF THE INVENTION

The present inventors have surprisingly found that the SARS-CoV-2 spike protein interferes with the signalling pathways of estrogen and vitamin D receptors.

In details, the present inventors have found that the SARS-CoV-2 spike protein contains a LxxLL motif that is homolog to that of the nuclear coactivator 1 (NCOA1), and is able to bind to the NCOA1 binding domain both on the estrogen and vitamin D receptors, thus inducing a conformational change in each of these receptors that decreases their affinity for the ligand, ultimately blocking the activation of the receptors.

In a clinical context of COVID-19, this mechanism exacerbates a situation of reduced immune response due to low circulating levels of estrogen and Vitamin D, as supported by the clinical evidence discussed above. In the context of COVID-19 vaccination, the spike protein produced following vaccination, on the one hand triggers an immune response but, at the same time, decreases such response by blocking estrogen and Vitamin D receptors activity, thereby interfering with the efficacy of the vaccine. Furthermore, the inhibition of the estrogen and Vitamin D pathways is responsible for a number of side effects observed for the vaccines, such as, in particular fatigue and osteoarticular pain which may be due due to the exacerbation of a pre-existing situation of reduced immune response as above reported.

The inventors have also found that this effect can be reverted by increasing the concentration of agonists of estrogen or of vitamin D, so as to facilitate the binding to the respective receptors, thereby overcoming the block induced by the spike protein and inducing a conformational change that increases affinity in favour of the coactivator compared to the spike protein, with consequent reactivation of the receptor. This results in a protective effect on the mechanisms downstream of receptor activation. In view of the above, the administration of an agonist of estrogen receptor and/or of Vitamin D receptor in combination with the administration of a COVID-19 vaccine is expected to be beneficial in order to increase the immune response against the spike protein and decrease the side effects of vaccination.

Accordingly, a first object of the invention a combination of an estrogen receptor agonist and/or a vitamin D receptor agonist and a COVID-19 vaccine.

A further object of the invention is the combination of the invention for use in the prevention of COVID-19.

A further object if the invention is an estrogen agonist and/or vitamin D receptor agonist for use in combination with a COVID-19 vaccine for the prevention of COVID-19.

A further object of the invention is a COVID-19 vaccine for use in combination with an estrogen receptor agonist and/or vitamin D receptor agonist for the prevention of COVID-19.

A further object of the invention is a pharmaceutical composition comprising an estrogen receptor agonist and/or a vitamin D receptor agonist and pharmaceutically acceptable excipients, for use in combination with a COVID-19 vaccine for the prevention of COVID-19 and its side effects.

A further object of the invention is a pharmaceutical composition comprising an estrogen receptor agonist, a vitamin D receptor agonist and pharmaceutically acceptable excipients.

A further object of the invention is a kit of parts comprising:
a) a spike protein based COVID-19 vaccine, as described below; and
b) a pharmaceutical composition comprising an effective amount of an estrogen receptor agonist and/or a vitamin D receptor agonist, as described below.

A further object of the invention is a kit of parts comprising:
a) a spike protein based COVID-19 vaccine, as described below; and
b) a pharmaceutical composition comprising an effective amount of an estrogen receptor agonist, as described below, in admixture with pharmaceutically acceptable excipients, and
b') a pharmaceutical composition comprising an effective amount of a vitamin D receptor agonist, as described below, in admixture with pharmaceutically acceptable excipients. A further object if the invention is a method for the prevention of COVID-19 in a human being comprising the administration to said human being COVID-19 vaccine in combination with an estrogen receptor agonist and/or a vitamin D receptor agonist.

### DESCRIPTION OF THE FIGURES

Figure 1 shows the Estrogen Receptor (ER) complexed with nuclear receptor coactivator. The whole compex is reported in cartoon; the ER monomers are in gray surface, while the NCOA segments are reported in black cartoon and lines.
Figure 2 shows the network of the most significant interactions of ER1 and ER2 (both in red spheres). Among the most reliable interacting proteins, the Nuclear receptor COActivators (NCOAs) directly bind the nuclear receptors and stimulate transcriptional activities.
Figure 3 shows the sequence alignment among LDX region in spike (aminoacids 818-822) and other LDX cofactor-receptor domains.
Figure 4 shows the 3D best docking hypothesis for spike-ER blind docking. The proteins are reported in cartoon. The ER dimer is in white cartoon while the spike protein is reported in black surface.
Figure 5 shows the 3D best docking hypothesis for spike-ER motif-oriented docking. The proteins are reported in cartoon. The ER dimer is in white cartoon while the spike protein is reported in black surface.

### DETAILED DESCRIPTION OF THE INVENTION

A first object of the invention is a combination of an estrogen receptor agonist and/or a vitamin D receptor agonist and a COVID-19 vaccine.

A further object of the present invention is the above combination for use in the prevention of COVID-19.

A further object if the invention is a COVID-19 vaccine for use in combination with an estrogen receptor agonist and/or vitamin D receptor agonist for the prevention of COVID-19.

A further object if the invention is an estrogen agonist and/or vitamin D receptor agonist for use in combination with a COVID-19 vaccine for the prevention of COVID-19.

Said COVID-19 vaccine is a spike based vaccine. With the wording "spike based vaccine" it is meant any vaccine against COVID-19 that uses the spike protein of SARS-CoV-2 or an immunogenic portion thereof as an antigen. This includes any vaccine whose administration to a subject introduces into the body of such subject the SARS-CoV-2 spike protein of SARS-CoV-2 or an immunogenic portion thereof either as such or bound to the inactivated or live attenuated virus.

According to a preferred embodiment, said COVID-19 vaccine is a mRNA or DNA vaccine.

According to the present invention, the wording "mRNA or DNA vaccine" refers to any vaccine that comprises mRNA or DNA whose administration to a subject results into production into such subject of the spike protein or an immunogenic portion thereof. Said immunogenic portion of the spike protein preferably has an aminoacid sequence consisting of at least 50%, 60%, 70%, 80% or 90% of the aminoacid sequence and including aminoacids from 818 to 822 of the full length SARS-CoV-2 spike protein.

The wording "estrogen receptor agonist" according to the present invention refers to any compound that is able to bind to the estrogen receptor and activate its transcriptional activity.

Preferably, said estrogen receptor agonist is selected from the group consisting of:
- endogenous estrogens, preferably selected from 17β-estradiol, 17α-estradiol, estetrol, estrone and estriol;
- phytoestrogens, preferably selected from coumestrol, daidzein, equol, formononetin and genistein;
- Selective Estrogen Receptor Modulators (SERM), preferably selected from tamoxifen, raloxifene, 4-hydroxy tamoxifen, droloxifene, ospemifene, arzoxifene and bazedoxifene.

Particularly preferred estrogen receptor agonists according to the invention are 17β-estradiol and raloxifen.

The wording "vitamin D receptor agonist" according to the present invention refers to any compound that is able to bind to the vitamin D receptor and activate its transcriptional activity.

Preferably, said Vitamin D receptor agonist is selected from the group consisting of vitamin D3 (cholecalciferol), 1α,25-dihydroxyvitamin D3 (calcitriol), 1α-hydroxyvitamin D3 (Alfacalcidol), vitamin D2 (ergocalciferol), 19-nor-1α,25-dihydroxyvitamin D2 (paricalcitol) and 1α-hydroxyvitamin D2 (doxercalciferol) and mixtures thereof.

Particularly preferred Vitamin D receptor agonists according to the invention are vitamin D3, vitamin D2 and mixtures thereof.

As discussed above, the combination of an estrogen receptor agonist and/or a vitamin D receptor agonist with the COVID-19 vaccine enhances the immune response stimulated by the vaccine, which would otherwise be reduced or slowed down by the interaction of the spike protein with estrogen and/or vitamin D receptors and therefore allows to obtain a more efficient immunity against the SARS-CoV-2 infection. Furthermore, the above combination also prevents side effects resulting from the interference of the spike proteins produced in the host after administration of the COVID-19 vaccine with the physiological function of estrogen and vitamin D.

These findings result in an improved combination therapy for preventing COVID-19.

In the above combination, the estrogen receptor agonist and/or a vitamin D receptor agonist can be administered concurrently or separately, preferably they are administered separately from the spike protein based COVID-19 vaccine and with a different schedule of administration.

Preferably, according to the present invention, the combination comprises both the estrogen receptor agonist and the vitamin D receptor agonist.

According to an embodiment, the estrogen receptor agonist and the vitamin D receptor agonist, when both present, are administered separately one from the other.

According to an alternative embodiment, the estrogen receptor agonist and the vitamin D receptor agonist, when both present, are administered together.

Preferably, the estrogen receptor agonist and/or a vitamin D receptor agonist are administered to the subject receiving a COVID-19 vaccine for a period of time starting between two weeks before and the day of administration of the first dose of vaccine and ending at least two weeks after the administration of the last dose of vaccine.

The frequency of administration and duration of the treatment will vary depending on the dose of estrogen receptor agonist and/or a vitamin D receptor agonist used and also on the type of COVID-19 vaccine.

According to one embodiment, the above estrogen receptor agonist and/or a vitamin D receptor agonist are used at the dose and frequency usually employed for the treatment of other pathologies were the activation of the estrogen and/or vitamin D receptor is beneficial.

More preferably, in order to determine the correct dose and schedule of administration, the levels of estrogens and/or vitamin D in the subject due to receive the COVID-19 vaccine is measured before vaccination and the treatment with the agonist(s) planned on the basis of their basal levels in the patient.

The estrogen receptor agonist and/or a vitamin D receptor agonist are preferably administered to the subject receiving or due to receive the COVID-19 vaccine in form of a pharmaceutical composition. The pharmaceutical composition may contain either the estrogen receptor agonist or the vitamin D receptor agonist or contain both the agonists together.

Accordingly, a further object of the present invention is a pharmaceutical composition comprising an estrogen receptor agonist or a vitamin D receptor agonist as previously defined, and pharmaceutically acceptable excipients, for use in combination with a COVID-19 vaccine for the prevention of COVID-19.

A further object of the present invention is a pharmaceutical composition comprising an estrogen receptor agonist and a vitamin D receptor agonist as previously defined, and pharmaceutically acceptable excipients.

A further object of the present invention is a pharmaceutical composition comprising an estrogen receptor agonist and a vitamin D receptor agonist as previously defined, and pharmaceutically acceptable excipients, for use in combination with a COVID-19 vaccine for the prevention of COVID-19.

Preferably, the pharmaceutical composition of the present invention is prepared in suitable dosage forms comprising an effective amount of agonist(s) and pharmaceutically acceptable excipients.

The administration of the pharmaceutical composition of the present invention to a patient is in accordance with known methods and may comprise oral administration, parenteral administration, preferably selected from intravenous, intraperitoneal, intramuscular, intraarterial, subcutaneous administration, topical administration, buccal administration or rectal (suppository) administration.

According to the present invention, the wording "effective amount" means a dosage of a compound or composition sufficient to significantly achieve the desired response.

The vaccine and the estrogen receptor agonist and/or the vitamin D receptor agonist may be present as a kit of parts.

Accordingly, another object of the present invention is a kit of parts comprising:
a) the COVID-19 vaccine as described above, and
b) a pharmaceutical composition comprising an effective amount of the estrogen receptor agonist and/or a vitamin D receptor agonist, as described above, in admixture with pharmaceutically acceptable excipients.

Another object of the present invention is a kit of parts comprising
a) a COVID-19 vaccine as described above, and
b) a pharmaceutical composition comprising an effective amount of the estrogen receptor agonist as described above, in admixture with pharmaceutically acceptable excipients, and/or
b') a pharmaceutical composition comprising an effective amount of vitamin D receptor agonist as described above, in admixture with pharmaceutically acceptable excipients. The route of administration of the agonist compounds or pharmaceutical composition of the present invention depends on the specific agonist used or contained in the pharmaceutical composition and is in accordance with known methods for administration of the compound, which is usually by systemic administration, preferably by oral, parenteral or inhalatory route. The term parenteral as used herein includes intravenous, intraperitoneal, intracerebral, intrathecal, intracranial, intramuscular, intraarticular, intrasynovial, intrasternal, intraocular, intraarterial, subcutaneous, intracutaneous injection or infusion techniques.

The composition of the present invention may be formulated into oral, inhalatory or injectable dosage forms such as tablets, capsules, powders, solutions, suspensions, and emulsions.

The pharmaceutically acceptable excipient according to the present invention includesany and all solvents, diluents, or other vehicle, dispersion or suspension aids, surface active agents, isotonic agents, thickening or emulsifying agents, preservatives, solid binders, lubricants and the like, as suited to the particular dosage form desired. Some examples of materials which can serve as pharmaceutically acceptable excipient include, but are not limited to, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatine; talc; excipients such as cocoa butter and suppository waxes; oils such as peanut oil, cottonseed oil; safflower oil; sesame oil; olive oil; corn oil and soybean oil; glycols; such a propylene glycol; esters such as ethyl oleate and ethyl laurate; agar; buffering agents such as magnesium hydroxide and aluminum hydroxide; alginic acid; pyrogen-free water; isotonic saline; sterilized water; Ringer's solution; buffered saline; dextrose solution; maltodextrin solution; ethyl alcohol; and phosphate buffer solutions. Moreover, the composition of the present invention may be formulated into inhalable or injectable dosage forms such as solutions, suspensions, and emulsions by further adding diluents, dispersants, and surfactants.

Further, the composition of the present invention may be suitably formulated using appropriate methods known in the art or by the method disclosed in Remington's Pharmaceutical Science (recent edition), Mack Publishing Company, Easton Pa.

The terms "pharmaceutically acceptable" and "physiologically acceptable" are intended to define, without any limitation, any material suitable for preparing a pharmaceutical composition to be administered to a living being.

The dosage forms can also contain other traditional ingredients such as: preservatives, stabilizers, surfactants, buffers, osmotic regulators, emulsifiers, sweeteners, colorants, flavourings and the like.

The dosage forms of the pharmaceutical composition of the present invention can be prepared by techniques that are familiar to a pharmaceutical chemist, and comprise mixing, granulation, compression, dissolution, sterilization and the like.

### EXPERIMENTAL PART

### Materials and Methods

### 1. Interactome analysis

The STRING database [Szklarczyk D et al., Nucleic Acids Res. 2021, 49: D605-D612, doi: 10.1093/nar/gkaa1074], that integrate all known and predicted associations between proteins, including both physical interactions as well as functional associations was used to analyse functional associations between biomolecules. Each protein-protein interaction is annotated with a 'score'. This score does not indicate the strength or the specificity of the interaction, but only the confidence. All scores rank from 0 to 1, with 1 being the highest possible confidence.

### 2. 3D Model selection

Spike 3D model was built based on PDB 6VYB returned to its wild-type form and fully glycosylated. An asymmetric glycosylation of the three protomers was derived by glycoanalyitic data for the N-glycans and O-glycans as published (Casalini L et al, ACS Cent. Sci. 2020, 6:1722-1734, https://doi.org/10.1021/acscentsci.0c01056). The proteins were modeled using Amber14SB force field (Maier J A et al, J. Chem. Theory Comput. 2015, 11:3696-3713, https://doi.org/10.1021/acs.jctc.5b00255) and the carbohydrate moieties by the GLYCAM06j-1 version of GLYCAM06 force field (Kirschner KN et al, J. Comput. Chem. 2008, 29:622-655, https://doi.org/10.1002/jcc.20820). The so prepared structure was used as starting point for Molecular Docking simulations. Topology files were generated with the pdb2gmx GROMACS tool, using the amber99sb forcefield (Lindorff-Larsen K et al, Proteins 2010,78:1950-1958, https://dx.doi.org/10.1002/prot.22711). Protein was inserted in a triclinic box, extending up to 15 Å from the solute, and immersed in TIP3P water molecules (Jorgensen WL et al, J Chem Phys 1983, 79:926-935, https://dx.doi.org/10.1063/1.445869). Counter ions were added to neutralize the overall charge with the genion GROMACS tool. After energy minimizations, the system was relaxed for 5 ns by applying positional restraints of 1000 kJ mol-1 nm-2 to the protein atoms. Following this step, unrestrained MD simulation was carried out for a length of 1 microsecond, with a time step of 2 fs, using GROMACS 2018.3 simulation package (supercomputer Galileo and Marconi-100, CINECA, Bologna, Italy) (Abraham MJ et al., SoftwareX 2015, 1:19-25, https://dx.doi.org/10.1016/j.softx.2015.06.001). V-rescale temperature coupling was employed to keep the temperature constant at 300 K (Bussi G et al, J Chem Phys, 2007 126:014101. https://dx.doi.org/10.1063/1.2408420). The Particle-Mesh Ewald method was used for the treatment of the long-range electrostatic interactions (Darden T et al., J. Chem. Phys 1993, 98:10089, https://doi.org/10.1063/1.464397). The first 5 ns of each trajectory were excluded from the analysis. The trajectory obtained after 1 microsecond MD simulation was clustered in order to obtain representative structures. In particular, the structure used for the docking studies is the first centroid of the first cluster extracted from the MD experiment.

For the estrogen receptor, the XRAY PDB model with code 3OLL was used, containing Estradiol and Nuclear receptor coactivator 1 (Möcklinghoff S et al, ChemBioChem 2010, 11:2251-2254, https://doi.org/10.1002/cbic.201000532).

### 3. Protein-Protein docking procedure

The input of two individual proteins, one for the receptor and the other for the ligand, were provided. In particular, the Spike protein and ER were used as receptor and ligand, respectively. Then, the HDOCK tool performs docking to sample putative binding modes through an FFT-based search method, and then scoring the protein-protein interactions. Finally, the top 100 predicted complex structures were provided, and the best ten hypotheses were visually inspected to confirm the reliability of the calculation. The entire workflow is described (Yan Y et al, Nat Protoc 2020, 15:1829-1852, https://doi.org/10.1038/s41596-020-0312-x).

Given the lack of structural information on the binding mechanism between the Spike protein and the nuclear estrogen receptor (ER), the first check involved the identification of proteins known to interact with estrogen receptors. After identifying these ER interacting proteins, the second step involved the study of any sequence analogies between the spike protein and the ER effector proteins.

### Example 1

### Nuclear receptor coactivators and LxxLL motif

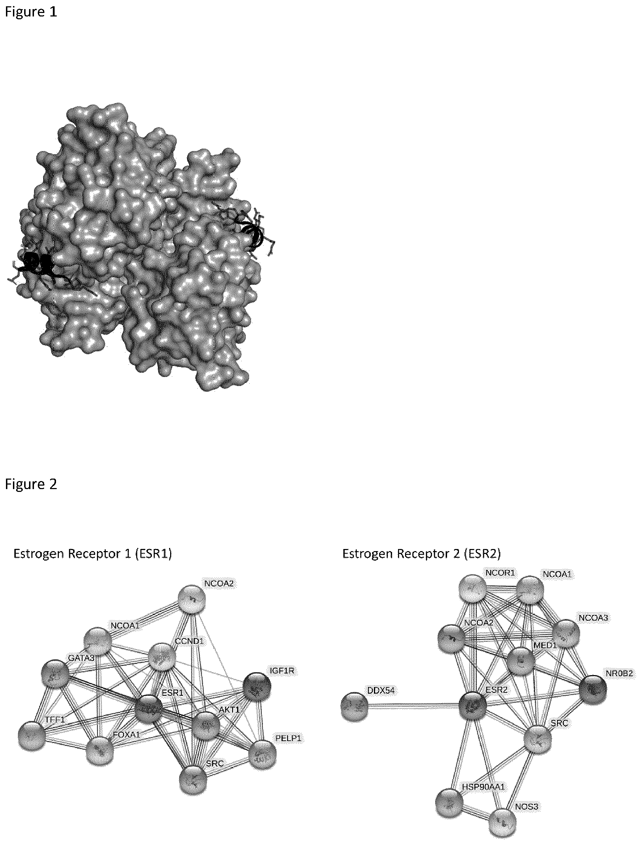

Many transcription factors and co-factors exhibit a common structural motif that ensures interaction with effector proteins. The motif that participates in these protein-protein interactions is termed LxxLL (LDX), and it is associated with different aspects of transcriptional regulation (Plevin MJ et al, Trends Biochem Sci 2005, 30:66-69. https://dx.doi.org/10.1016/j.tibs.2004.12.001). The LxxLL sequence was originally identified in proteins that bind the activation function-2 (AF-2) region of nuclear receptor ligand-binding domains (LBDs). These motifs are fundamentals in nuclear-receptor regulation with many nuclear-receptor-binding proteins, including co-activators (NCOA-1, 2 and 3) (Heery DM et al, Nature 1997, 387:733-736, https://doi.org/10.1038/42750). The experimental and structural confirmations which show the interaction between ER and NCOAs, made it possible to focus on the LxxLL motif and starting from this, a mapping of the spike protein sequence was carried out searching for structural motifs and homologous portions of spike able to mimic the interaction between the ER and its nuclear co-activators (Figure 1).

### Example 2

### Estrogen receptor binding protein network analysis

The network of most significant interactions of ER1 and ER2 (both in the spheres) (Figure 2) highlights thata, among the most reliable interacting proteins with ER1 and ER2, the Nuclear receptor COActivators (NCOAs) directly bind nuclear receptors and stimulate transcriptional activities in a hormone-dependent fashion. Many experimental data of complexes between ER and NCOAs deposited in the Protein Data Bank, support these findings, and can be found in the Uniprot database (ER1: https://www.uniprot.org/uniprot/P03372#structure; ER2: https://www.uniprot.org/uniprot/Q92731#structure).

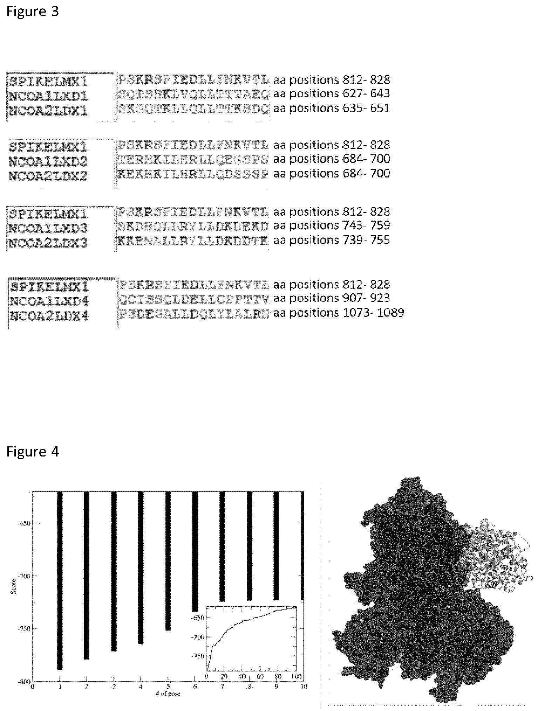

Combining the sequence alignment between NACOs and spike with a three-dimensional analysis of the viral protein, it emerged that the spike protein contains a sequence homologous to LxxLL, also valid from a structural point of view (in fact, this regions assumes alpha-helix conformations), in an outer zone and which could, in principle, act as interacting region site with ER (Figure 3, aminoacids 818 to 828 of SARS-CoV-2 spike proteins). Different LDX domain are associated to specific cofactor-receptor interaction and the spike LDX1 resemble NCOA1 LDX4 and belong to Class III domain (Savkur RS et al, J Pept Res 2004, 63:207-212, https://dx.doi.org/10.1111/j.1399-3011.2004.00126.x)

### Example 3

### Spike protein-ER blind docking

It has been experimentally verified that ER does not bind the Receptor Binding Domain (RBD) of the viral protein (data not shown). To confirm and validate the in silico prediction, the ability of ER to interact in a region other than the viral RBD was evaluated by means of a blind-docking between the two proteins, using the HDOCK server [http://hdock.phys.hust.edu.cn/]. The blind-docking approach does not consider any structural bias and is fully unguided. The best binding hypothesis found puts in evidence a high affinity of ER towards the lateral region of the spike protein, belonging to the so called "fusion peptide portion" (Figure 4).

### Example 4

### Spike protein-ER motif-oriented docking

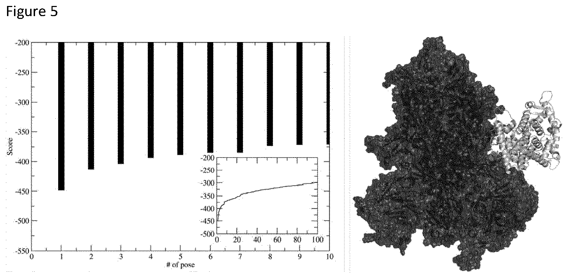

The structura information that ER residues are recognized by NCOAs was used to guide the docking study of ER on spike by optimizing protein-protein interactions. With the protein structures and residue restraints as input, a suitable model was generated by the HDOCK server. Given that the structural information of interaction between ER and NCOA are known, a second docking study was carried out, that considered the ER residues that guarantee interaction with the NCOAs, thus guiding the molecular docking procedure. The best binding hypothesis obtained from the guided docking study is shown in Figure 5 that highlights the binding of the ER to the spike region containing the motifs homologous to the LxxLL pattern (Figure 5).

### Discussion

Based on the same computational approach, several studies conducted on the interaction between Vitamin D and its receptor and between spike protein and Vitamin D receptor, gave results very similar to those obtained with studies on the estrogen receptor, highlighting also for Vitamin D receptor, the presence of coactivators needed for activation, and the presence of the LxxLL motif that is conserved also for the Vitamin D receptor. The results of the docking studies show also in this case the binding of the vitamin D receptor to the region of the spike protein that contains the motif homologous to the LxxLL pattern.

In any case, since vitamin D has multiple cellular and intracellular targets, additional studies are needed to determine the consequences of the interaction of Vitamin D in the immune-response against SARS-CoV-2 in order to achieve a significant vision into new prophylactic and therapeutic strategies to prevent this viral infection.

## Claims

1. A combination of an estrogen receptor agonist and/or a vitamin D receptor agonist and a COVID-19 vaccine.

2. A combination according to claim 1, for use in the prevention of COVID-19.

3. A combination as claimed in claims 1 and 2, wherein said COVID-19 vaccine uses the spike protein of SARS-CoV-2 or an immunogenic portion thereof as an antigen.

4. A combination as claimed in claims 1 to 3, wherein said COVID-19 vaccine is a mRNA or DNA vaccine.

5. A combination as claimed in claims 1 to 4, wherein said estrogen receptor agonist is selected from the group consisting of:
- endogenous estrogens, preferably selected from 17β-estradiol, 17α-estradiol, estetrol, estrone and estriol;
- phytoestrogens, preferably selected from coumestrol, daidzein, equol, formononetin and genistein;
- Selective Estrogen Receptor Modulators (SERM), preferably selected from tamoxifen, raloxifene, 4-hydroxy tamoxifen, droloxifene, ospemifene, arzoxifene and bazedoxifene.

6. A combination as claimed in claims 1 to 5, wherein said estrogen receptor agonist is 17β-estradiol or raloxifen.

7. A combination as claimed in claims 1 to 6, wherein said vitamin D receptor agonist is selected from the group consisting of vitamin D3 (cholecalciferol), 1α,25-dihydroxyvitamin D3 (calcitriol), 1α-hydroxyvitamin D3 (Alfacalcidol), vitamin D2 (ergocalciferol), 19-nor-1α,25-dihydroxyvitamin D2 (paricalcitol) and 1α-hydroxyvitamin D2 (doxercalciferol) and mixtures thereof.

8. A combination as claimed in claims 1 to 7, wherein said vitamin D receptor agonist is vitamin D3, vitamin D2 or a mixture thereof.

9. A combination as claimed in claims 1 to 8, comprising both the estrogen receptor agonist and the vitamin D receptor agonist.

10. A combination as claimed in claims 1 to 9, wherein said estrogen receptor agonist and/or a vitamin D receptor agonist are administered to a subject receiving said COVID-19 vaccine for a period of time starting between two weeks and the day of administration of the first dose of vaccine and ending at least two weeks from the administration of the last dose of vaccine

11. A pharmaceutical composition comprising an estrogen receptor agonist and/or a vitamin D receptor agonist and/or pharmaceutically acceptable excipients, for use in combination with a COVID-19 vaccine for the prevention of COVID-19.

12. A pharmaceutical composition comprising an estrogen receptor agonist and a vitamin D receptor agonist and pharmaceutically acceptable excipients.

13. A kit of parts comprising:
a) a COVID-19 vaccine, as described in any of the previous claims; and
b) a pharmaceutical composition comprising an effective amount of estrogen receptor agonist and/or a vitamin D receptor agonist, as described in any of the previous claims.

14. A kit of parts comprising:
a) a COVID-19 vaccine, as described in any of the previous claims; and
b) a pharmaceutical composition comprising an effective amount of estrogen receptor agonist, as described in any of the previous claims, in admixture with pharmaceutically acceptable excipients, and
b') a pharmaceutical composition comprising an effective amount of vitamin D receptor agonist, as described in any of the previous claims, in admixture with pharmaceutically acceptable excipients.
